# EUROPÄISCHE PATENTSCHRIFT

(11) **EP 0 867 175 B1**
(45) Veröffentlichungstag und Bekanntmachung des Hinweises auf die Patenterteilung: **09.08.2000**
(21) Anmeldenummer: 98104984.4
(22) Anmeldetag: 19.03.1998
(51) Int. Cl.: A61K 7/48, A61K 47/48

(54) **Verfahren zur Stabilisierung von Retinol bzw. Retinol-Derivaten**
Process of stabilisation of retinol or its derivatives
Procédé de stabilisation du rétinol ou de ses dérivés

(30) Priorität: 27.03.1997 DE 19713092
(43) Veröffentlichungstag der Anmeldung: 30.09.1998
(73) Patentinhaber: Wacker-Chemie GmbH, 81737 München (DE)
(72) Erfinder: Moldenhauer, Jens-Peter, Dr., 84489 Burghausen (DE); Regiert, Marlies, 80538 München (DE); Wimmer, Thomas, Dr., 84533 Marktl (DE)
(74) Vertreter: Potten, Holger

(56) Entgegenhaltungen:
- EP-A- 0 335 545
- EP-A- 0 399 716
- EP-A- 0 579 435
- EP-A- 0 657 176
- WO-A-82/00251
- WO-A-91/11172
- JP-A- 2 108 622
- US-A- 4 371 673
- US-A- 5 472 954
- DATABASE WPI Week 9022 Derwent Publications Ltd., London, GB; AN 90-167901 XP002070791 & JP 02 108 622 A (NISSHIN FLOUR MILLING CO.) , 19.Oktober 1988
- CHEMICAL ABSTRACTS, vol. 126, no. 7, 17.Februar 1997 Columbus, Ohio, US; abstract no. 98643, MUNOZ BOTELLA ET AL.: "analytical applications of retinoid-cyclodextrin inclusion complexes" XP002070790 & ANALYST, Bd. 121, Nr. 11, 1996, CAMBRIDGE,UK, Seite 1557-1560

## Beschreibung

Die Erfindung betrifft Verfahren zur Stabilisierung von Retinol bzw. Retinol-Derivaten gegenüber Oxidations- und UV-Angriffen.

Cyclodextrine sind cyclische Oligosaccharide, die aus 6,7 oder 8 α(1-4)-verknüpften Anhydroglukoseeinheiten aufgebaut sind. Die durch enzymatische Stärkekonversion hergestellten α-, β- oder γ-Cyclodextrine unterscheiden sich in dem Durchmesser ihrer hydrophoben Kavität und eignen sich generell zum Einschluß zahlreicher lipophiler Substanzen.

Retinol (Vitamin A) ist ein fettlösliches, aus einem Cyclohexen-Ring mit einer konjugiert mehrfach ungesättigten Seitenkette aufgebautes Vitamin. Es ist u.a. für den Menschen essentiell und spielt besonders für den Sehvorgang eine wichtige Rolle. Der tägliche Bedarf wird fast ausschließlich über die Aufnahme von β-Carotin (Pro-Vitamin A) gedeckt, welches enzymatisch in Retinol umgewandelt wird.

Retinol ist aufgrund seiner wichtigen physiologischen Funktion, vor allem beim Sehvorgang, sowie seiner hautpflegenden und faltenvermindernden Eigenschaften eine äußerst wertvolle Substanz. Im Bereich der Kosmetik besteht ein großes Interesse am verstärkten Einsatz von Vitamin A, besonders in dermatologischen Formulierungen.

Die topische Applikation von Retinol stabilisiert den Vitamin A-Haushalt der Haut, der insbesondere durch die Bestrahlung mit UV-Licht nachhaltig gestört sein kann. Eine Unterversorgung mit Vitamin A führt zu einer Schädigung speziell der Epidermis, zu verstärkter Falten- und Hornbildung ("Photoaging") und zu einem Elastizitätsverlust der Haut. Darüber hinaus wird ihre Barrierefunktion gegenüber Mikroorganismen geschwächt.

Das Hauptproblem für die breitere Anwendung von Retinol besteht in seiner Empfindlichkeit gegen Oxidation, insbesondere unter Lichteinwirkung. An der konjugiert ungesättigten Seitenkette des Moleküls findet eine Autoxidation statt, die zur Bildung zahlreicher Zersetzungsprodukte, zu Isomerisierungen und Polymerisationen führt. Aus der ursprünglich kristallinen Substanz entsteht dabei eine zähe Masse, die hellgelbe Farbe des reinen Retinols wird intensiver. Durch intermediär gebildete Peroxide erhöht sich das toxische Potential der Formulierungen, die kosmetisch erwünschten Effekte des intakten Retinols werden verringert.

Anstelle des sehr empfindlichen freien Retinols werden daher in der Kosmetik, speziell in Anti-Falten-Cremes, vielfach die weniger wirksamen, aber stabileren Retinylester, insbesondere Retinylacetat und -palmitat, eingesetzt.

Darüber hinaus sind Retinol-haltige Vitamin-Präparate im Lebensmittelbereich auf dem Markt. Weiterhin kann Retinol in pharmazeutischen Formulierungen verwendet werden.

Möglichkeiten zur Stabilisierung des reinen Retinols sind somit von außerordentlichem Interesse. Wegen der extremen Empfindlichkeit des freien Retinols gegen oxidative Zersetzung ist sein an sich erwünschter Einsatz stark eingeschränkt.

In dem US-Patent 2,827,452 wird gezeigt, daß Retinol sowie Retinylacetat und -palmitat mit Hilfe von β-Cyclodextrin stabilisiert werden können. Ein α-Cyclodextrin/Retinol-Komplex wird in CA: 98:52237 als Zusatzstoff in der Lebensmitteltechnologie empfohlen.

In CA: 110:199059 wird ein die Photostabilität von Retinylacetat erhöhender Effekt von β-Cyclodextrin und β-Cyclodextrin-Derivaten in wäßriger Lösung und im festen Zustand beschrieben.

Aus CA: 120:265222 ist die Komplexbildung von Retinylacetat mit β-Cyclodextrin bekannt.

In WO 94/21225 ist eine hautpflegende Formulierung mit Retinylpalmitat und β-Cyclodextrin beschrieben.

In WO 90/14082 wird Retinsäure zusammen mit β-Cyclodextrin in einem wäßrigen Gel zu dermatologischen Zwecken eingesetzt.

In US 5,484,816 wird die Stabilisierung von Vitamin A und entsprechender Fettsäureester, die auch in dermatologischen Formulierungen vorkommen können, mit Hilfe von Antioxidantien und UV-Absorbern, die als Cyclodextrin-Komplexe vorliegen, offenbart.

In J. Drug Targeting 2(5) (1994) 449-54 (CA: 122:64224) wird über die Komplexierung von Retinol und Retinsäure mit Hydroxypropyl-β-Cyclodextrin und den Einschluß der entsprechenden Komplexe in Liposomen berichtet.

In US 5,024,998 wird gezeigt, daß nach parenteraler Anwendung das Risiko einer unerwünschten Ansammlung von Retinol als lipophilem pharmazeutischem Wirkstoff durch Solubilisierung mit Hilfe von Hydroxypropyl-β-Cyclodextrin vermindert werden kann.

WO 82 00251 A (US COMMERCE) 4. Februar 1982 offenbart Komplexe aus γ-Cyclodextrin und Retinolsäure. Der Komplex wird zur Verbesserung der Wirkstoffsolubilisierung verwendet, um den toxischen Effekt des Wirkstoffs herabzusetzen.

DATABASE WPI, Week 9022 Derwent Publications Ltd., London, GB; AN 90-167901, XP002070791 & JP 02 108 622 A (NISSHIN FLOUR MILLING CO.), 19. Oktober 1988 beschreibt einen Komplex enthaltend α-Tocopherol-Vitamin A-Säureester und γ-Cyclodextrin. Der Komplex dient einer guten Solubilisierung von α-Tocopherol-Vitamin A-Säureester sowie seiner Stabilisierung gegen Hitze.

CHEMICAL ABSTRACTS, vol. 126, no. 7, 17. Februar 1997 Colum- bus, Ohio, US; abstract no. 98643, MUNOZ BOTELLA ET AL.: "analytical applications of retinoid-cyclodextrin inclusion complexes" XP002070790 & ANALYST, Bd. 121, Nr. 11, 1996, CAMBRIDE, UK, Seite 1557-1560 beschreibt ebenfalls einen Komplex aus γ-Cyclodextrin und Retinol oder Retinol-Derivat.

Trotz der zitierten vielfach bekannten Möglichkeit der Verwendung von β-Cyclodextrin bzw. β-Cyclodextrin-Derivaten zum Einschluß und zur Stabilisierung von Retinol und Retinol-Derivaten wurden in letzter Zeit zur Erhöhung der Stabilisierung von Retinol in Formulierungen eher Cyclodextrin-Komplexe von Stabilisatorsubstanzen der Formulierung zugesetzt.

Dies zeigt, daß der Fachmann die Möglichkeit der Stabilitätserhöhung durch Verwendung von Cyclodextrinen oder Cyclodextrinderivaten als ausgeschöpft betrachtete und andere Wege zur Stabilitätserhöhung des Retinols oder seiner Derivate suchte.

Die Erfindung betrifft Verfahren zur Stabilisierung von Retinol oder Retinol-Derivat gegenüber Oxidations- und UV-Angriffen dadurch gekennzeichnet, daß Retinol oder ein Retinol-Derivat mittels γ-Cyclodextrin komplexiert wird, wobei die Komplexierung in einer wäßrigen Cyclodextrinlösung mit einer Cyclodextrin-Konzentration zwischen 5 - 50 Gewichts-% erfolgt oder die Komplexierung durch Kneten einer wäßrigen Cyclodextrin-Paste mit einer Cyclodextrin-Konzentration zwischen 25 - 80 Gewichts-% erfolgt.

Unter Retinol-Derivaten sind im Sinn der Erfindung Retinylester (Vitamin A-Ester) und Vitamin A-Säure zu verstehen.

Vorzugsweise sind unter Retinol-Derivaten Retinylester zu verstehen.

Im Folgenden soll der Begriff Retinol auch Retinol-Derivate mit umfassen.

Überraschend zeigte sich, daß sich Retinol hervorragend durch γ-Cyclodextrin stabilisieren läßt. Die erfindungsgemäßen Verfahren stabilisieren Retinol gegenüber oxidativer Zersetzung und UV-Angriffen stärker als bekannte Verfahren.

Als vorteilhaft hat sich die Herstellung der erfindungsgemäßen Komplexe aus konzentrierten, wäßrigen γ-Cyclodextrin-Lösungen erwiesen.

Alternativ ist es möglich, daß die Komplexierung durch Kneten einer wäßrigen Cyclodextrin-Paste mit einer Cyclodextrin-Konzentration zwischen 25 - 80 Gewichts-% erfolgt.

Die Cyclodextrin-Konzentration der wäßrigen Lösung liegt zwischen 5 - 50 Gewichts-%. Bevorzugt ist eine Cyclodextrin-Konzentration von 20- 50 %.

Das Gewichtsverhältnis Retinol zu Cyclodextrin liegt zwischen 1 : 20 und 1 : 1, bevorzugt zwischen 1: 12 und 1 : 4.

Die Ansätze werden intensiv vermischt, d.h. je nach Konsistenz intensiv gerührt oder geknetet.

Die Reaktionstemperatur liegt vorzugsweise bei 20 - 80°C. Besonders bevorzugt wird bei 20 - 60 °C, insbesondere bevorzugt bei 40 - 60 °C gearbeitet.

Die Reaktionsdauer hängt von der Temperatur ab und liegt zwischen einer Stunde und einigen Tagen. Bevorzugt ist eine Reaktionszeit von 12 bis 96 Stunden.
Die Komplexierung erfolgt vorzugsweise unter Normaldruck.

Bevorzugt findet die Komplexierung unter Schutzgasatmosphäre (z. B. Stickstoff oder Argon) statt.

Die wenig wasserlöslichen Komplexe können direkt in Form der Reaktionsmischung verwendet werden. Sie können aber auch durch Filtration, Zentrifugation, Trocknung, Mahlen, Sieben, Sichten, Granulieren, Tablettieren entsprechend den jeweils üblichen Verfahren isoliert und aufbereitet werden.

Je nach Einsatzzweck z.B. in kosmetischen Formulierungen können noch weitere Substanzen den γ-Cyclodextrin-Komplexen zugesetzt werden. So können z.B. Tenside, waschaktive, pflegende, selbstbräunende Zusätze, Verdickungsmittel, Konservierungsmittel, Stabilisatoren, Emulgatoren, Duftstoffe, Farbstoffe, Antioxidantien, Vitamine, UV-Filter, Silikonöle zugesetzt werden.

Die folgenden Beispiele dienen der weiteren Erläuterung der Erfindung:

### Beispiel 1: Komplexierung von Retinol mit β- bzw. γ-Cyclodextrin für einen Vergleich der Lagerstabilität

a) (Vergleichsbeispiel) 78.5 g β-Cyclodextrin wurden in 1520 ml abgekochtem und Stickstoff-gesättigtem dest. Wasser bei 55 °C gelöst und unter Schutzgasatmosphäre mit 10 g Retinol versetzt. Bei dieser Temperatur wurde der Ansatz 72 h gerührt. Nach dem Abkühlen auf Raumtemperatur wurde der Komplex durch Filtration isoliert und im Vakuum getrocknet. Die Ausbeute betrug 79.2 g.
b) (erfindungsgemäßes Beispiel) 1097 g γ-Cyclodextrin wurden in einem thermostatisierten Planschliffgefäß mit 1900 ml dest. Wasser verrührt, auf 90°C aufgeheizt und unter Stickstoff auf 50°C abgekühlt. Nach der Zugabe von 100 g Retinol wurde der Ansatz bei 50°C 72 h intensiv gerührt und anschließend auf Raumtemperatur abgekühlt. Der entstandene Komplex wurde abgesaugt und im Vakuum getrocknet. Ausbeute: 1125 g.

### Beispiel 2: Herstellung eines Retinol/γ-Cyclodextrin-Komplexes nach der Knetmethode

370 g γ-Cyclodextrin wurden in einer Knetmaschine mit 280 ml abgekochtem und Stickstoff-gesättigtem dest. Wasser zu einer Paste verrührt. Dann wurden unter Schutzgas 40 g Retinol zugegeben. Die Mischung wurde auf 50 °C aufgeheizt und bei dieser Temperatur unter weiterer Wasserzugabe 6h gerührt bzw. geknetet. Nach dem Abkühlen auf Raumtemperatur wurde das Produkt entnommen und der Komplex durch Trocknen im Vakuum gewonnen.

### Beispiel 3: Vergleichsversuche zur Bestimmung der Lagerstabilität von Retinol als β- und γ-Cyclodextrin-Komplex

Jeweils 50 g der Komplexe von Retinol mit β- und γ-Cyclodextrin (nach Beispiel 1a-b) wurden in flache Petrischalen gefüllt und bei Raumtemperatur mit UVA-Licht der Wellenlänge 366 nm bestrahlt. Durch Umrühren wurden die Proben vor der Gehaltsbestimmung homogenisiert. In Tabelle 1 sind die mittels HPLC-Analyse bestimmten Retinolgehalte aufgeführt. Der stabilisierende Effekt ist beim γ-Cyclodextrin-Komplex deutlich stärker ausgeprägt als beim β-Cyclodextrin-Komplex.

**Tabelle 1:**

| Lagerzeit [Tage] | Retinolgehalt | | | |
|---|---|---|---|---|
| | Retinol-β-CD-Komplex | | Retinol-γ-CD-Komplex | |
| | absolut | relativ | absolut | relativ |
| 0 | 7.1 % | 100 % | 8.9 % | 100 % |
| 4 | 1.7 % | 23.9 % | 5.0 % | 56.2 % |
| 7 | 1.0 % | 14.1 % | 3.6 % | 40.5 % |
| 48 | 0.0 % | 0.0 % | 1.6 % | 18.0 % |

### Beispiel 4: Vergleich eines γ-Cyclodextrin-Komplexes mit einer Lactoseverreibung von Retinol

4a: 45 g γ-Cyclodextrin wurden bei 50°C in 100 ml dest. Wasser gelöst. Nach Zugabe von 5 g Retinol wurde die Reaktionsmischung unter Schutzgas 48 h gerührt und der ausgefallene Komplex durch Filtration isoliert und im Vakuum getrocknet. Ausbeute: 47 g mit einem Vitamingehalt von 10%.

4b: 45 g β-D-Lactose wurden in einer Reibschale intensiv mit 5 g Retinol verrieben, bis ein homogenes Pulver mit einem Vitamingehalt von 10% erhalten wurde.

Je 40 g des γ-Cyclodextrin-Komplexes (4a) und der Lactoseverreibung (4b) wurden in offenen Petrischalen bei Raumtemperatur und Tageslicht gelagert. Im zeitlichen Abstand von 4,7,21 und 48 Tagen nach Lagerbeginn wurden Proben der Substanzen 4a und 4b gezogen und der Gehalt an noch intaktem Retinol mittels HPLC-Analyse bestimmt. Tabelle 2 zeigt die erhaltenen Werte.

**Tabelle 2:**

| Lagerzeit [Tage] | Retinolgehalt | | | |
|---|---|---|---|---|
| | Retinol-Lactoseverreibung | | Retinol-γ-CD-Komplex | |
| | absolut | relativ | absolut | relativ |
| 0 | 8.8 % | 100 % | 8.9 % | 100 % |
| 4 | 5.4 % | 61:4 % | 8.2 % | 92.1 % |
| 7 | 3.5 % | 39.8 % | 7.8 % | 87.6 % |
| 21 | 1.4 % | 15.9 % | 6.4 % | 71.9 % |
| 48 | 0.2 % | 2.3 % | 5.6 % | 62.9 % |

### Beispiel 5: Herstellung eines Retinylacetat/γ-Cyclodextrin-Komplexes nach der Knetmethode

250 g γ-Cyclodextrin wurden in einer Knetmaschine mit 160 ml abgekochtem und Stickstoff-gesättigtem dest. Wasser zu einer Paste verrührt. Dann wurden unter Schutzgas 63 g Retinylacetat untergemischt. Die Paste wurde auf 55 °C aufgeheizt und bei dieser Temperatur unter weiterer Wasserzugabe 8h geknetet. Nach dem Abkühlen auf Raumtemperatur wurde das Produkt entnommen und der Komplex durch Trocknen im Vakuum gewonnen.

### Beispiel 6: Gesichtspackung in Pulverform (zur Selbstbereitung)

Zusammensetzung: Gewichtsteile:
1) Kaolin 300
2) Mandelkleie (gesiebt) 145
3) γ-Cyclodextrinkomplex mit 25% Nachtkerzenöl 550
4) γ-Cyclodextrinkomplex mit 9.8% Retinol 5

### Zubereitung der Puderformulierung:

Die Bestandteile von 1-4 werden über einen kombinierten Misch-, Mahl- und Siebprozeß zu einem Puder formuliert.

### Zubereitung der Gesichtspackung:

2 Eßlöffel des Puders werden mit warmem Wasser zu einem Brei verrührt und genügend dick, doch streichbar, auf die Haut aufgebracht. Nach 15 Minuten wird warm abgewaschen.

### Beispiel 7: Körperemulsion

Zusammensetzung: Gewichtsteile:
1) Glycerinmonomyristat 14
2) Stearinsäure 12
3) Cetylalkohol 5
4) Isopropylpalmitat 50
5) γ-Cyclodextrin-Komplex mit 9.8% Retinol 4
6) Wasser dest. 905
7) Methylparaben 10

### Zubereitung:

Die Rohstoffe 1-5 werden in einem Becherglas vorgelegt, die Rohstoffe 6 und 7 in einem Rührgefäß gemischt und auf 65°C erwärmt. Beide Mischungen emulgiert man bei 65°C mit einem schnellaufenden Flügelrührer. Unter weiterem Rühren läßt man auf 40°C abkühlen und homogenisiert mit dem Ultra-Turrax (max. 500 UpM). Die in der Creme gelöste Luft muß durch vorsichtiges Anlegen von Wasserstrahlvakuum entfernt werden.

### Beispiel 8: Antifalten-Creme

Zusammensetzung: Gewichtsteile:
1) Wasser dest. 650
2) γ-Cyclodextrin 100
3) Macadamianußöl 190
4) Jojobaöl 30
5) Avocadoöl 20
6) γ-Cyclodextrin-Komplex mit 9.8% Retinol 10

### Zubereitung:

Die Rohstoffe 1 und 2 werden in einem Becherglas vorgelegt und auf 50°C erwärmt. Das Macadamianußöl wird zugegeben und die Mischung bei hoher Rührgeschwindigkeit 2 Stunden homogenisiert. Unter weiterem Rühren läßt man abkühlen, fügt die Rohstoffe 4 -6 zu und homogenisiert mit dem Ultra-Turrax (max. 500 UpM) weitere 10 Minuten.
Die Viskosität der Creme hat nach einer Lagerzeit von 5 Tagen das Optimum erreicht.

## Patentansprüche

1. Verfahren zur Stabilisierung von Retinol oder Retinol-Derivaten gegenüber Oxidations- und UV-Angriffen, dadurch gekennzeichnet, daß Retinol oder Retinol- Derivat mittels γ-Cyclodextrin komplexiert wird, wobei die Komplexierung in einer wäßrigen Cyclodextrinlösung mit einer Cyclodextrin-Konzentration zwischen 5 - 50 Gewichts-% erfolgt oder die Komplexierung durch Kneten einer wäßrigen Cyclodextrin-Paste mit einer Cyclodextrin-Konzentration zwischen 25 - 80 Gewichts- % erfolgt.

2. Verfahren gemäß Anspruch 1, dadurch gekennzeichnet, daß das Gewichtsverhältnis Retinol zu Cyclodextrin zwischen 1 : 20 und 1 : 1 liegt.

3. Verfahren gemäß einem der Ansprüche 1 oder 2, dadurch gekennzeichnet, daß das Gewichtsverhältnis Retinol zu Cyclodextrin zwischen 1 : 12 und 1 : 4 liegt.

4. Verfahren gemäß einem der Ansprüche 1 bis 3, dadurch gekennzeichnet, daß die Reaktionstemperatur bei 20 - 80°C liegt.

5. Verfahren gemäß einem der Ansprüche 1 bis 4, dadurch gekennzeichnet, daß die Komplexierung unter Schutzgasatmosphäre stattfindet.

## Claims

1. Process for stabilizing retinol or retinol derivatives with respect to oxidation and UV attacks, characterized in that retinol or a retinol derivative is complexed using γ-cyclodextrin, the complexation reaction being carried out in an aqueous cyclodextrin solution having a cyclodextrin concentration of between [sic] 5 - 50% by weight, or the complexation reaction being carried out by kneading an aqueous cyclodextrin paste having a cyclodextrin concentration of between [sic] 25 - 80% by weight.

2. Process according to Claim 1, characterized in that the weight ratio of retinol to cyclodextrin is between 1 : 20 and 1 : 1.

3. Process according to either of Claims 1 or 2, characterized in that the weight ratio of retinol to cyclodextrin is between 1 : 12 and 1 : 4.

4. Process according to one of Claims 1 to 3, characterized in that the reaction temperature is 20 - 80°C.

5. Process according to one of Claims 1 to 4, characterized in that the complexation reaction is carried out under a protective gas atmosphere.

## Revendications

1. Procédé de stabilisation du rétinol ou de dérivés de rétinol vis-à-vis d'attaques par oxydation et par UV, caractérisé en ce que le rétinol ou un dérivé de rétinol est complexé au moyen de γ-cyclodextrine, la complexation s'effectuant dans une solution de cyclodextrine aqueuse avec une concentration en cyclodextrine entre 5-50% en poids ou la complexation s'effectuant par pétrissage d'une pâte aqueuse de cyclodextrine avec une concentration en cyclodextrine entre 25-80% en poids.

2. Procédé suivant la revendication 1, caractérisé en ce que le rapport pondéral du rétinol à la cyclodextrine se situe entre 1:20 et 1:1.

3. Procédé suivant l'une des revendications 1 ou 2, caractérisé en ce que le rapport pondéral du rétinol à la cyclodextrine se situe entre 1:12 et 1:4.

4. Procédé suivant l'une des revendications 1 à 3, caractérisé en ce que la température réactionnelle se situe à 20-80°C.

5. Procédé suivant l'une des revendications 1 à 4, caractérisé en ce que la complexation a lieu sous atmosphère de gaz protecteur.
